**Europäisches Patentamt**

**European Patent Office**

**Office européen des brevets**

(19)

(11) Veröffentlichungsnummer: **0 378 095**

**A1**

(12) # EUROPÄISCHE PATENTANMELDUNG

(21) Anmeldenummer: **90100095.0**

(22) Anmeldetag: **03.01.90**

(51) Int. Cl.5: **A61B 17/34**

(30) Priorität: **07.01.89 DE 3900329**

(43) Veröffentlichungstag der Anmeldung:
**18.07.90 Patentblatt 90/29**

(84) Benannte Vertragsstaaten:
**AT BE CH DE DK ES FR GB GR IT LI NL SE**

(71) Anmelder: **B. Braun Melsungen AG**
**Carl-Braun Strasse**
**D-3508 Melsungen(DE)**

(72) Erfinder: **Heise, Peter**
**Heiligenbergstrasse 23**
**D-3501 Fuldabrück(DE)**
Erfinder: **Gerlach, Roland**
**Kirchweg 9**
**D-3501 Guxhagen(DE)**
Erfinder: **Keller, Hans-Jörg**
**Zeller Strasse 109**
**D-7600 Offenburg(DE)**
Erfinder: **Haacke, Claus**
**Altstadt 6**
**D-3508 Melsungen(DE)**
Erfinder: **Vahlensieck, Winfried, c/o Klinikum**
**Grosshadern**
**Urologische Abteilung, Marchioninistrasse**
**15**
**D-8000 München 70(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Besteck zur Punktion eines Körperhohlraumes.**

(57) Das Besteck zur Punktion eines Körperhohlraumes, insbesondere zur suprapubischen Harnblasendrainage, besteht aus einer längsteilbaren Hülse (11), in der ein herausziehbarer, zylindrischer Trokar (12) steckt, dessen Endstück (13) über den distalen Rand der Hülse (11) vorsteht und mit einer dreikantig angeschliffenen, geschlossenen Spitze (41) versehen ist. Erfindungsgemäß ist der distale Rand (14) der Hülse (11) gegen den zylindrischen Umfang (20) des Trokars (12) abfallend angefast und es ist die angeschliffene Spitze (41) am distalen Ende einer konischen Dilatierzone (40) des Endstückes (13) ausgebildet. Der Trokar (12) ist hohl. Hinter der Dilatierzone (40) und vor dem distalen Rand (14) der Hülse (11) weist er ein in den Trokarlängskanal (22) mündendes Seitenloch (19) auf und ist am proximalen Ende mit einem Auslaß versehen. Ein solches Besteck zeichnet sich durch niedrige Punktionskräfte und durch vermindertes Verletzungsrisiko für die Gefäße der Körperhohlraumwand bei der Penetration aus.

FIG. 1

## Besteck zur Punktion eines Körperhohlraumes

Die Erfindung bezieht sich auf ein Besteck zur Punktion eines Körperhohlraumes, insbesondere zur suprapubischen Punktion der Harnblase, bestehend aus einer längsteilbaren zylindrischen Hülse, in der ein herausziehbarer Trokar steckt, dessen massives Endstück über den distalen Rand der Hülse vorsteht und mit einer dreikantig angeschliffenen Spitze versehen ist.

Zur suprapubischen Blasendrainage ist es erforderlich, die Harnblase durch die Bauchdecke zu punktieren, um einen Zugang zur Katheterableitung des Urins zu besitzen, wenn der transurethrale Weg nicht gewünscht ist. Als Punktionsinstrumente sind mehrere Bestecke bekannt, deren Gemeinsamkeit darin besteht, daß sie eine Hülse aufweisen, die bei der Punktion in den Körperhohlraum vordringt und durch die in den Körperhohlraum ein Katheter einführbar ist, der die Körperflüssigkeit ableitet. Ein solches bekanntes Besteck besteht gemäß DE-A-21 04 211 aus einer zylindrischen Stahlkanüle, deren distales Ende zur Bildung einer Spitze abgeschrägt und durch Facettenschliff mit zwei scharfen Schneiden versehen ist. Um die Stahlkanüle nach Einführung eines Katheters mit an seinem proximalen Ende unlösbar befestigten Katheteransatz, z.B. einem Anschlußkonus, von dem Katheter abnehmen zu können, ist diese bekannte Stahlkanüle mit axialen Trennlinien versehen, so daß sie durch Angriff einer seitwärts gerichteten Kraft entlang den als Sollbruchstellen wirkenden Trennlinien aufgetrennt und seitlich von dem Katheter abgezogen werden kann. Sowohl bei diesem Besteck als auch bei einer Blasenpunktionskanüle gemäß DE-A- 33 47 150, deren abgeschrägtes distales Ende über den halben Kreisumfang facettenartig scharf angeschliffen ist und die aus zwei längsgeschlitzten, koaxialen, zueinander verdrehbaren zylindrischen Hülsen gebildet ist, sind niedrige Punktionskräfte erforderlich. Dies ist an sich vorteilhaft, jedoch erhöhen die messerartigen scharfen Schneiden bei der Punktion die Häufigkeit behandlungsbedürftiger Blutungskomplikationen, weil durch die scharf geschliffene Punktionshohlnadel bei Auftreffen auf ein Blutgefäß ein Schneideffekt mit konsekutiver Blutung auftritt.

Zur Verkleinerung des Einschnittes in Körpergewebe ist bei einem Punktionsbesteck zur Kathetereinführung in ein Blutgefäß nach US-A- 4 565 545 in einer nicht teilbaren Hülse eine herausziehbare Kanüle angeordnet, deren über den Rand der Hülse vorstehender kegelstumpfförmig verjüngter Spitzenabschnitt einen die schräge Lumenöffnung der Kanüle umgebenden Facettenschliff aufweist. Der Randabschnitt der Hülse ist ebenfalls außen kegelförmig verjüngt und seine Stirnfläche bildet mit dem Außenumfang der Kanüle eine Ringstufe. In der Kanüle steckt ein Mandrin, dessen distale schräge Stirnfläche mit der Lumenöffnung der Kanüle bündig abschließt. Der kegelstumpfförmige Abschnitt der Kanüle dient der Aufweitung des durch den Facettenschliff erzeugten Schnittes in der Wand des Blutgefäßes. Dabei behindert die Ringstufe eine gleichmäßige Fortsetzung der Aufweitung durch den Randabschnitt der Hülse. Nach Herausziehen der Kanüle mit Mandrin aus der Hülse wird durch diese ein Katheter verlegt. Zur Schaffung eines Zuganges z.B. zur Harnblase ist dieses Punktionsbesteck we gen der messerartigen Facettenschliffspitze und der Verletzungsgefahr für Blutgefäßwandungen beim Durchtritt durch die Bauchdecke ebenfalls ungeeignet. Auch darf der Katheter keinen festgeklebten Katheteransatz aufweisen, weil die nicht spaltbare Hülse über das proximale Ende des Katheters nach hinten abgezogen werden muß. Ein aufgesteckter oder eingesteckter Katheteransatz ist jedoch nachteilig, weil er Undichtigkeitsprobleme aufwirft. Ferner ist bei der Ableitung von Flüssigkeiten aus dem Körperhohlraum ein eingesteckter Katheteransatz ungünstig, weil seine ringförmige Stirnfläche in dem Katheter eine Kante bildet, die ein Abflußhindernis darstellt.

Auch sind Punktionsbestecke bekannt (DE-A- 22 04 664, US-A- 37 89 852), die in einer Hülse ein herausziehbares Punktionsinstrument mit geschlossener konischer Spitze aufweisen. In beiden Fällen ist der distale Rand der Hülse gegen den Umfang des Punktionsinstrumentes abfallend abgeschrägt. Beide Hülsen sind nicht durch Längsteilung öffenbar. Gewebeausstanzungen werden bei der Punktion zwar vermieden, jedoch sind hohe Punktionskräfte erforderlich.

Bei einem Besteck der eingangs erwähnten Art, das in der Zeitschrift "Urologe B" (1988), 28, Seiten 177/178 beschrieben ist, steckt in der zylindrischen Hülse mit schräger Lumenöffnung und Facettenanschliff ein über seine ganze Lange zylindrischer Trokar, dessen zylindrisches Endstück aus der Hülse ragt und durch dreikantigen Anschliff der geschlossenen Spitze bis zum Außenrand der zylindrischen Umfangsfläche des Trokars verlaufende lange Schneiden erhält. Die drei scharfen Schliffe des Trokars reduzieren zwar die Punktionskräfte, können aber trotzdem das getroffene Blutgefäß durch Schnittwirkung verletzen, so daß die Blutungshäufigkeit unerwünscht hoch bleibt.

Der Erfindung liegt die Aufgabe zugrunde, die bekannten Punktionsbestecke so zu verbessern, daß sie sich durch niedrige Punktionskräfte und durch vermindertes Verletzungsrisiko für die Gefäße der Körperhohlraumwand bei der Penetration auszeichnen.

Diese Aufgabe wird erfindungsgemäß dadurch gelöst, daß der dreikantige Anschliff an der Spitze einer konischen Dilatierzone des Endstückes des Trokars ausgebildet ist, die aus dem bis an den zylindrischen Umfang des Trokars abfallend angefasten distalen Rand der Hülse hervorragt.

Die drei Schneiden der geschlossenen Spitze des Trokars sind infolge der nach vorne konisch verjüngten Dilatierzone kürzer als bei einem Trokar mit dem Durchmesser seines zylindrischen Teiles entsprechend bemessener Spitze und sie sind deshalb beim Vordringen der Spitze in den Körperhohlraum beim Auftreffen auf ein Blutgefäß ungefährlicher. Die Schneiden passieren das Blutgefäß schnell und die konische Dilatierzone drängt es sofort vorsichtig zur Seite und von den Schneiden weg. Dieser vorteilhafte Effekt wird durch die gegen den zylindrischen Umfang des Trokars abfallende Abfasung des distalen Randes der Hülse unterstützt, der einen sanft ansteigenden Übergangsbereich zwischen Außenfläche des Endstückes des Trokars und der Außenfläche der Hülse schafft, welcher das durchquerte Gewebe auch an dieser Stelle schont. Die "Entschärfung" der Schneiden durch die konische Dilatierzone mit kreisförmigem Querschnitt und der stufenlose Übergang zwischen Trokar und Hülse ermöglichen einen problemlosen Einsatz des Bestecks durch erleichterte Punktion und Handhabung mit verringertem Blutungsrisiko.

In vorteilhafter Ausgestaltung der Erfindung ist vorgesehen, daß der Trokar hohl ist, hinter der Dilatierzone und vor dem distalen Rand der Hülse ein in den Trokar längskanal mündendes Seitenloch aufweist und am proximalen Ende mit einem Auslaß versehen ist. Diese Ausbildung dient der Sichtbarmachung des Punktionserfolges, d.h. der Penetration der Harnblase oder eines anderen Körperhohlraumes, durch am proximalen (patientenfernen) Ende austretenden Urin bzw. anderes Körperfluid. Das Seitenloch befindet sich in der zylindrischen Umfangszone des über den distalen Rand der Hülse vorstehenden Endstückes des Trokars. Damit das Seitenloch von der Hülse frei bleibt, ist vorgesehen, daß bei senkrecht zur Längsachse verlaufendem angefasten Rand der Hülse dieser von der konischen Dilatierzone ausreichend weit zurückgesetzt ist. Um das von der Hülse freie Endstück des Trokars aus Gründen erleichterter Führung des Bestecks bei dem Penetrationsvorgang zu verkürzen, ist es zweckmäßig, daß der angefaste Rand der Hülse schräg zur Längsachse verläuft und Hülse und Trokar so zueinander ausgerichtet sind, daß der vorstehende Teil des Randes etwa an die konische Dilatierzone heranreicht und der nach hinten versetzte Teil des Randes das Seitenloch freiläßt. Die korrekte Position von Hülse und Trokar in bezug aufeinander und ihre Blockierung gegen axiale gegenseitige Verschiebung wird durch eine Arretiervorrichtung gesichert. Diese ist vorteilhafterweise im Bereich eines Griffstückes des Trokars und eines Griffteiles der Hülse vorgesehen. Sie kann als Lock-Verriegelung ausgebildet und so eingerichtet sein, daß der Trokar und die Hülse sich während der Punktion nicht zueinander verdrehen oder verschieben können, wodurch die Schliffe des angefasten Randes der Hülse und der Spitze des Trokars in vorgegebener Weise zueinander orientiert bleiben. Der Auslaß des Trokarlängskanals steht mit einer transparenten Kammer des Griffstückes des Trokars in Verbindung. Die transparente Kammer kann als an eine Öffnung einer Auslaßleitung des Griffstückes angesetzter transparenter Schlauch ausgebildet sein, der der Anzeige des Fluidrückstromes dient. Alternativ kann zu diesem Zweck das Griffstück hohl und aus transparentem Material ausgebildet sein. Zum Ablassen von Fluid wird ein Verschluß, z.B. ein hydrophober Stopfen, aus einer Öffnung des Griffstückes oder dem Ende des Schlauches, herausgezogen.

Bei einer weiteren günstigen Ausführungsform der Erfindung ist der Trokar massiv und besitzt in seiner Außenfläche mindestens eine längsverlaufende Kerbe, die sich in dem zylindrischen Teil des Trokars von der Dilatierzone bis zu einem Auslaß am proximalen Ende erstreckt. An dem Übergang zwischen konischer Dilatierzone und zylindrischem Teil des Trokars ist jene Kerbe offen. Der Auslaß der Trokarkerbung steht mit einer transparenten Kammer des Griffteiles der Hülse in Verbindung. Auch in diesem Falle kann mit einer Öffnung einer Auslaßleitung des Griffteiles ein transparenter Schlauch verbunden sein, dessen Endöffnung durch einen Stopfen verschließbar ist. Zur Sichtbarmachung des Fluidrückflusses kann alternativ das Griffteil der Hülse hohl und aus transparentem Material ausgebildet sein. Dabei ist eine durch einen hydrophoben Stopfen verschlossene Öffnung in dem Griffteil zum Fluidablaß öffenbar.

Zur Abdichtung der Hülse gegenüber dem Trokar bzw. einem nach dessen Herausziehen einzuführenden Katheter ist vorteilhafterweise das längsteilbare Griffteil der Hülse mit einem Dichtelement ausgerüstet. Als Dichtelement kann eine scheibenförmige Membran oder ein Hütchen aus Elastomermaterial dienen. Die proximale Stirnfläche des Hütchens bzw. die scheibenförmige Membran können geschlossen oder geschlitzt sein. In beiden Fällen durchdringt die angeschliffene Spitze des Trokars die Dichtung und wird am proximalen Ende der längsteilbaren Hülse von ihr abdichtend umgeben. Das Dichtelement verhindert, daß Körperflüssigkeit zwischen Trokar und Hülse nach außen strömt, so daß die Handhabung für den Anwender hygienisch sicherer wird. Körperfluid strömt allein durch den Trokarlängskanal oder die Trokarkerbung in Richtung des Auslasses, in dessen Bereich sie in der transparenten Kammer sichtbar wird. Wenn nach erfolgreicher Punktion der Trokar aus der Hülse herausgezogen wird, schließt das Dichtelement den Kanal der Hülse und öffnet sich erst wieder unter dem Andruck eines durch die Hülse in den Körperhohlraum einzuführenden Katheters. Wenn der Katheter das Lumen des Körperhohlraumes erreicht hat, wird die Hülse über den Katheter zurückgezogen und zur seitlichen Abnahme von diesem aufgespalten. Um mit der Hülse auch das Dichtelement seitlich von dem Katheter entfernen zu können, sind vorzugsweise die Membran oder das Hütchen ebenfalls längs mindestens einer Schwächungslinie teilbar ausgebildet.

In einer weiteren Ausführungsform nach Anspruch 13 ist vorgesehen, daß das Hütchen einen etwa im Winkel von 45° zur Längsachse geneigten seitlichen schlauchförmigen Ansatz besitzt. Dieser Ansatz dient zur Aufnahme eines Katheterschlauches während der Punktion des Körperhohlraumes. Auf diese Weise kann nach Entfernen des inneren Trokars, nachdem also das Dichtelement die Durchtrittstelle des Trokars abgedichtet hat, auch der Katheter vorgeschoben werden, ohne daß Flüssigkeit ins Freie tritt.

In der Zeichnung sind Ausführungsbeispiele der Erfindung schematisch veranschaulicht. Es zeigen:

Fig. 1 das vordere, distale Ende eines Bestecks zur Punktion eines Körperhohlraumes in Draufsicht,

Fig. 2 einen Schnitt längs der Linie II-II in Fig. 1,

Fig. 3 das vordere distale Ende einer anderen Ausführungsform eines Bestecks zur Punktion eines Körperhohlraumes in Draufsicht,

Fig. 4 einen Schnitt längs der Linie IV-IV in Fig. 3,

Fig. 5 eine Draufsicht auf das komplette Besteck nach Fig. 1,

Fig. 6 eine Draufsicht auf das komplette Besteck nach Fig. 3,

Fign. 7 und 8 Draufsichten auf verschiedene Dichtelemente am proximalen Ende von erfindungsgemäßen Bestecks,

Fign. 9 bis 13 Draufsichten, Seitenansichten und Schnitte an verschiedenen Stellen eines weiteren Dichtelementes.

Ein Besteck 10 zur Punktion eines Körperhohlraumes, insbesondere zur suprapubischen Harnblasendrainage, besteht im wesentlichen aus einer kreiszylindrischen Hülse 11 aus Metall oder Polymermaterial oder einer Kombination beider Materialien und aus einem über den Hauptteil seiner Länge kreiszylindrischen Trokar 12 aus Metall, der im wesentlichen spaltfrei in der Hülse 11 steckt und aus dieser axial herausziehbar ist. In zusammengestecktem Zustand, d.h. im Benutzungszustand, steht ein distales Endstück 13 über den distalen Rand 14 der Hülse 11 vor. Der Rand 14 der Hülse 11 verläuft schräg zur Längsachse von Hülse 11 und Trokar 12, wobei der Abschrägungswinkel so gewählt ist, daß keine Spitze entsteht. Er ist ringsum von außen nach innen flach abfallend angefast, so daß er in bezug auf einen über den Rand 14 vorstehenden, kurzen Überstand 20 des zylindrischen Teiles des Trokars 12 nach hinten eine konische Erweiterung bildet. Der Winkel zwischen der Außenfläche des Überstandes 20 des Trokars und der gegen diese abfallenden Anfasung des Randes 14 beträgt etwa 120° bis 160°, vorzugsweise jedoch 140°. Wichtig ist, daß der Übergang zwischen der zylindrischen und der schrägen Fläche am Übergang zwischen Trokar 12 und Hülse 11 stufenlos ist, d.h. die Kante 14a des Randes 14 ist scharf. Bei einer aus Metall gefertigten Hülse 11 wird die Anfasung durch einen Randanschliff erzielt. Die Hülse 11 ist längsteilbar ausgebildet. Zu diesem Zweck sind zwei in Richtung ihrer Längsachse verlaufende, durch Schwächung (Einkerbung) der Hülsenwand entstandene, symmetrisch angeordnete Trennlinien 15 in der Hülse 11 ausgebildet, die sich von einem Ende zum anderen Ende der Hülse 11 erstrecken und als Sollbruchstellen wirken. Jeder Längshälfte 11a,11b der Hülse 11 ist eine seitwärts gerichtete Griffplatte 16,17 an einem mit der Hülse 11 längs der Trennlinien 15 teilbaren Hülsenansatz 18 zugeordnet, der als Griffteil dient. Nach Beendigung eines Punktionsvorganges und Verlegung eines Katheters durch die Hülse 11 wird diese aus dem Punktionsloch herausgezogen und mit dem Hülsenansatz 18 durch Angriff einer seitwärts gerichteten Kraft mit Hilfe der Griffplatten 16,17 entlang den Trennlinien 15 aufgespalten, so daß der Katheter von dem Besteck 10 befreit werden kann.

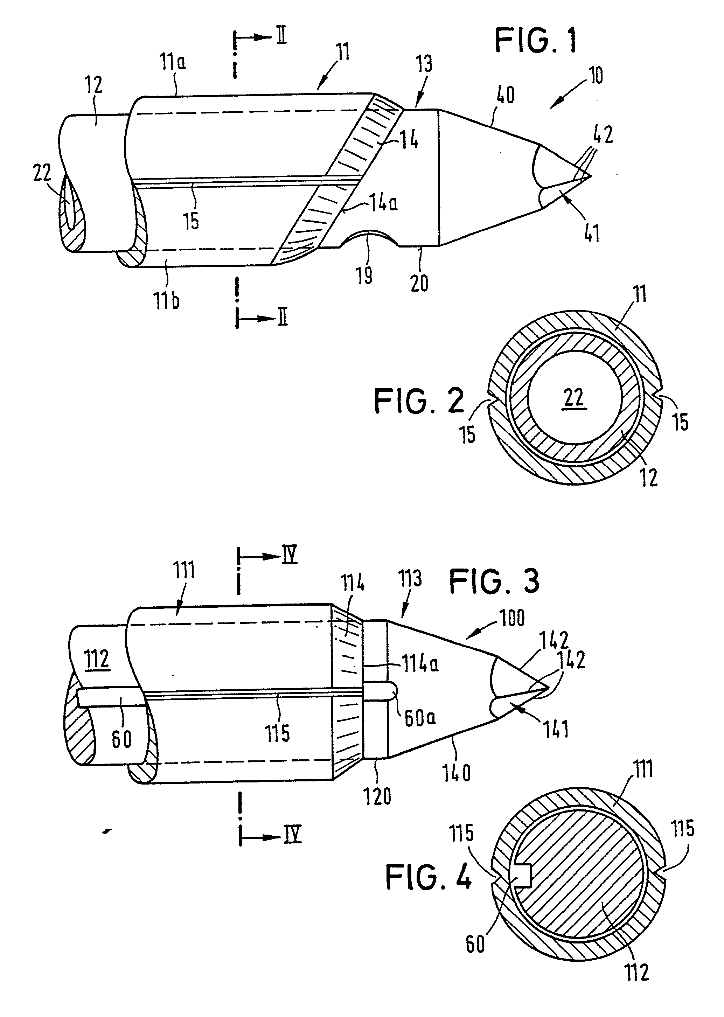

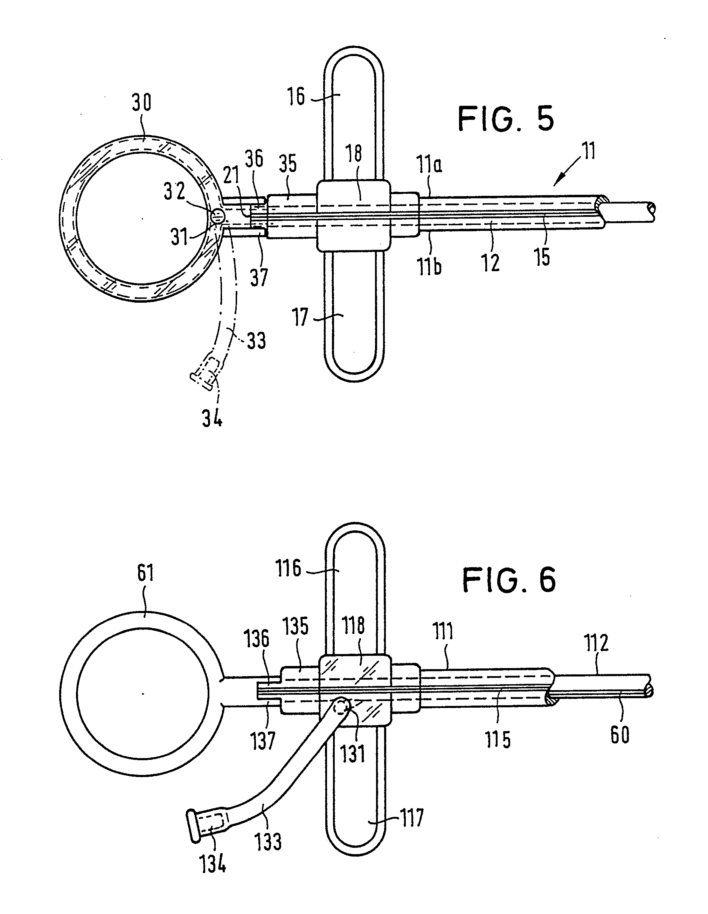

Bei dem Beispiel der Figuren 1 und 2 ist der Trokar 12 auf dem langen Stück mit kreiszylindrischem Umfang hohl ausgebildet, so daß er einen Längskanal 22 enthält. Der Längskanal 22 mündet an dem zylindrischen Teil des Endstückes 13 in einem Seitenloch 19, das infolge der Abschrägung des Randes 14 der Hülse 11 und entsprechender Orientierung der Hülse 11 auf dem Trokar 12 von der Hülse 11 frei ist, so daß es einen offenen seitlichen Einlaß zum Längskanal 22 bildet. Am hinteren proximalen Ende des Trokars 12 ist ein Auslaß 21 des Längskanals 22 ausgebildet, der aus einer endständigen Öffnung bestehen kann, die offen verbunden ist ist mit dem Hohlraum eines hohlen ringförmigen Griffstückes 30 aus transparentem Material (Fig. 5). In der Wand des Griffstükkes 30 ist vorzugsweise in der Nähe des Auslasses 21 des Trokars 12 eine Öffnung 31 ausgebildet, die von einem hydrophoben Stopfen 32 verschlossen ist. Wenn bei erfolgreicher Punktion eines Körperhohlraumes, z.B. der Harnblase, Urin durch das Seitenloch 19 und den Längskanal 22 des Trokars 12 in das transparente Griffstück 30 abfließt, erkennt der Anwender den Punktionserfolg unmittelbar durch Beobachtung des Griffstückes 30. Zum Ablassen des Urins aus dem Griffstück 30 wird der hydrophobe Stopfen 32 aus der Öffnung 31 herausgezogen.

Bei nichttransparenter Ausbildung des Griffstückes 30 kann zur Bildung einer transparenten Kammer der Auslaß 21 über einen kurzen Kanal mit der Öffnung 31 verbunden sein, und es kann in die Öffnung 31 ein transparenter Schlauch 33 fest eingeklebt oder angespritzt sein, dessen Ende durch einen hydrophoben Stopfen 34 verschlossen ist. Diese alternative Ausbildung einer transparenten Kammer als Punktionskontrollmöglichkeit ist in Figur 5 gestrichelt angedeutet.

Der Hülsenansatz 18 aus Kunststoff ist an seinem der Hülse 11 abgewandten Ende mit einer koaxialen Verlängerung 35 versehen, die mit dem Hülsenansatz 18 und der Hülse 11 längsteilbar ist und die ein Element einer Arretiervorrichtung zur Zusammenhaltung und Drehsicherung von Trokar 12 und Hülse 11 aufweist. Dabei kann es sich um eine übliche Lock-Verriegelung handeln, deren radiale Nasen 36 mit Rastorganen 37 an dem Griffstück 30 des Trokars 12 zusammenwirken. Zur übersichtlicheren Darstellung sind in den Fign. 5 und 6 bzw. 7 und 8 nur die Verriegelungselemente bzw. nur die Dichtungselemente eingezeichnet. Wie sich jedoch aus den Anwendungsbeispielen ergibt, ist das gleichzeitige Anbringen dieser beiden Elemente zweckmäßig und notwendig.

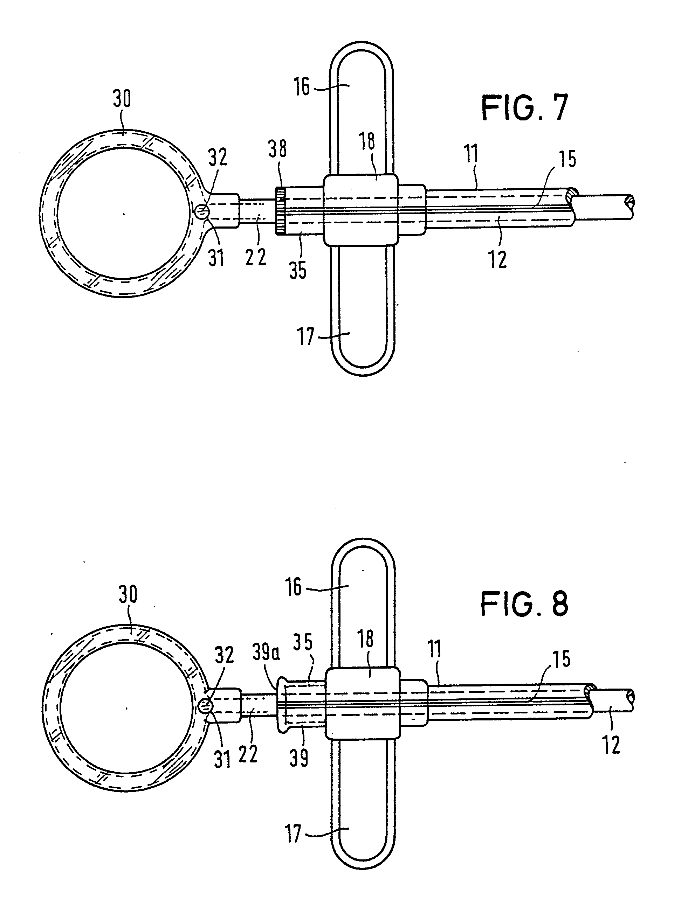

An den Rand des zylindrischen Teiles des Endstückes 13 des Trokars 12 schließt sich stufenlos eine konische Dilatierzone 40 an, deren distales verjüngtes Ende in einer dreikantig angeschliffenen, geschlossenen Spitze 41 endet. Die drei kurzen Kanten der Spitze 41 bilden scharfe Schneiden 42, die die Funktion des Gewebes mit geringem Kraftaufwand ermöglichen. Die unmittelbar anschließende Dilatierzone 40 weitet den Durchlaß auf und drängt in den Weg des Trokars 12 gelangende Blutgefäße vorsichtig zur Seite, so daß sie nicht angeritzt werden und die Blutungshäufigkeit (Makrohaematurie direkt nach der Punktion) gesenkt wird. Nach dem Durchdringen von Haut und Blasenwand kann das Abfließen des Urins durch den Längskanal 22 in dem transparenten hohlen Griffstück 30 oder dem transparenten Schlauch 33 erkannt werden. Anschließend wird der Trokar zurückgezogen. Dabei wird ein Dichtelement 38 (Figur 7) oder 39 (Figur 8) aktiv, um den axialen Durchlaß in dem Hülsenansatz 18 zu verschließen. Sodann wird ein Katheter mit fest angebrachtem Katheteransatz durch den Hülsenansatz 18 und die Hülse 11 in die Blase eingeführt.

Das Dichtelement 38 ist eine scheibenförmige Membran aus Elastomermaterial, die von dem Trokar 12 erst kurz vor der Punktion durchstochen wird und am äußeren Ende der Verlängerung 35 des Hülsenansatzes 18 angeordnet ist. Das Dichtelement 39 gemäß Figur 8 ist als Hütchen aus Elastomermaterial ausgebildet, das die Verlängerung 35 des Hülsenansatzes 18 überkappt und durch dessen membranartige Stirnwand 39a der Trokar 12 gestochen wird. Beide Dichtelement 38 und 39 sind selbstschließend. Beide können zum erleichterten Durchlaß des Trokars 12 bzw. des Katheters ein oder mehrfach geschlitzt sein. Auch ist es vorteilhaft, sie durch eine Schwächungslinie längsteilbar auszubilden, so daß sie bei Abnahme der Hülse 11 und des Hülsenansatzes 18 von dem Katheter mit festgeklebtem Katheteransatz von dem Katheter seitlich entfernt werden können. Die Trennlinien 15 sind zur schematischen Veranschaulichung der Trennbarkeit von Hülse 18, Verlängerung 35 und Dichtelement 38,39 durchgezogen worden.

Das Beispiel der Figuren 3, 4 und 6 unterscheidet sich von dem ersten Beispiel des Bestecks dadurch, daß das Besteck 100 einen massiven Trokar 112 aufweist, der zur Flüssigkeitsableitung in seiner Außenfläche mindestens eine achsparallele Kerbe 60 aufweist. Die Kerbe beginnt vorne bei 60a an dem über den Rand 114 der Hülse 111 vorstehenden Endstück 113 auf der Anschlußkante zwischen dem zylindrischen Umfangsteil 120 und der konischen Dilatierzone 140, deren geschlossene Spitze 141 durch Dreikantschliff drei scharfe Schneiden 142 erhält, die sich in einer scharfen Spitze treffen. Die Dilatierzone 140 hat kreisförmigen Querschnitt und verjüngt sich gegen die Spitze 141. Der Rand 114 der kreiszylindrischen Hülse 111, in der der Trokar 112 im wesentlichen spaltfrei und herausziehbar steckt, verläuft rechtwinklig zur Längsachse des Bestecks 100 und ist gegen den zylindrischen Teil 120 des Trokars 112 abfallend angefast. Die Kante 114a ist scharf, so daß ein stufenloser Übergang zwischen dem zylindrischen Teil 120 und dem angefasten Rand 114 erreicht wird. Die Kante 114a kann bündig mit dem Beginn der konischen Dilatierzone 140 verlaufen oder zu diesem - wie gezeigt - etwas zurückgesetzt sein. Die vordere Mündung der Kerbe 60 bleibt in jedem Falle offen, weil sie am Ende größten Durchmessers der konischen Dilatierzone 140 beginnt und damit eine axial gerichtete Öffnung 60a erhält. Auch bei diesem Beispiel kann die Hülse 111 aus Metall oder Polymermaterial oder einer Kombination beider Materialien bestehen und der Trokar 112 ist aus Metall hergestellt.

Die Hülse 111, der Hülsenansatz 118 und seine koaxiale Verlängerung 135 sind längs zwei paralleler eingekerbter Trennlinien 115 spaltbar. Der Hülsenansatz 118 am proximalen Ende der Hülse 111 trägt seitlich gerichtete Griffplatten 116,117, mit deren Hilfe er gemeinsam mit der Hülse 111 spaltbar ist, wie zu dem vorangegangenen Beispiel beschrieben wurde. Die Kerbe 60 des Trokars 112 endet an einem in Figur 6 angedeuteten Auslaß in dem als Griffteil dienenden Hülsenansatz 118, der mit einer Öffnung 131 in Verbindung steht. Wenn der Hülsen ansatz 118 hohl und transparent ausgebildet ist, so daß der Urinrückstrom durch die Kerbe 60 im Hülsenansatz 118 erkennbar ist, wird die Öffnung 131 mit einem hydrophoben Stopfen verschlossen. Zur Kenntlichmachung des Urinrückflusses dient anderenfalls ein in der Öffnung 131 befestigter transparenter Schlauch 133, dessen Ende durch einen hydrophoben Stopfen 134 verschlossen ist.

Der Trokar 112 ragt mit seinem kerbenfreien proximalen Ende durch die Verlängerung 135 des Hülsenansatzes 118 hindurch, die mit einem Dichtelement ähnlich der Scheibe 38 oder dem Hütchen 39 bzw. 45 ausgestattet ist, und eine Arretiervorrichtung hält den Trokar 112 und die Hülse 111 in vorgegebener Orientierung verdreh- und verschiebungssicher fest. Zu diesem Zweck sind an einem massiven Ring des Griffstückes 61 des Trokars 112 Rastorgane 137 vorgesehen, die mit Kupplungsteilen 136 der Verlängerung 135 lösbar zusammengreifen.

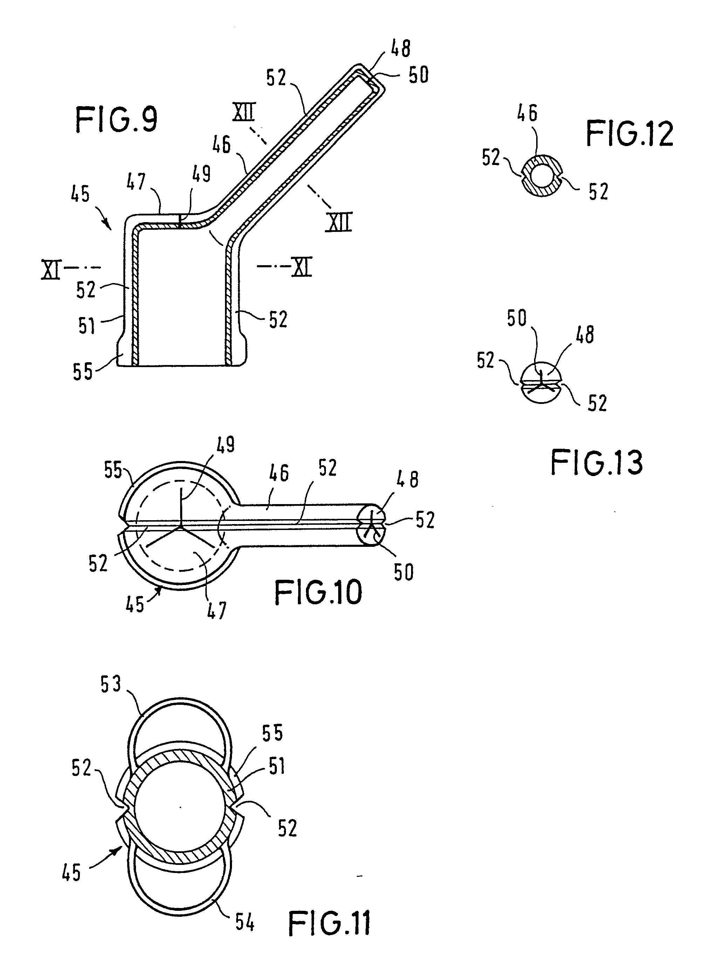

In den Fign. 9-13 sind Einzelheiten einer weiteren Ausführungsform eines Dichtelementes dargestellt, das als Hütchen 45 aus Elastomermaterial ausgebildet ist und als Ersatz der Dichtelemente 38 (Fig. 7) oder 39 (Fig. 8) dienen kann. Das Hütchen 45 wird mit seiner kreiszylindrischen Umfangswand 51, die in einer Ringwulst 55 endet, auf die Verlängerung 35 bzw. 135 des Hülsenansatzes 18 bzw. 118 festsitzend aufgeschoben. Die ebene Stirnwand 47 des Hütchens 45 ist mit einer Schlitzung 49 versehen, die aus einem zentralen geraden Schlitz oder aus den gezeichneten sternförmigen Schlitzen gebildet sein kann, welche sich in der Mitte der Stirnwand 47 treffen und den Trokar 12 bzw. 112 durchlassen bzw. nach seinem Herausziehen den Durchlaß selbsttätig verschließen. Im Randbereich zwischen Um fangswand 51 und Stirnwand 47 des kappenförmigen Hütchens 45 ist ein schlauchförmiger Ansatz 46 ausgebildet, dessen freies Ende von einer Stirnwand 48 verschlossen ist. Der Ansatz 46 verläuft unter einem Winkel von etwa 45° zur Längsachse des Hütchens 45 schräg nach oben. Sein Innendurchmesser ist dem Außendurchmesser eines einzuführenden Katheterschlauches angepaßt. Die Stirnfläche 48 des Ansatzes 46 ist mit einer Schlitzung 50 versehen, die ebenfalls aus drei zentral zusammenstoßenden Schlitzen zusammengesetzt sein kann. Das Hütchen 45 mit dem Ansatz 46 sollen wie die Dichtelemente 38 (Fig. 7) und 39 (Fig. 8) zur seitlichen Abnahme von einem verlegten Katheterschlauch mit der Hülse 11; 111, dem Hülsenansatz 18; 118 und der koaxialen Verlängerung 35;135 längsteilbar sein. Zu diesem Zweck dient eine eingekerbte Trennlinie 52, die so über den Hütchenkörper und den Ansatz 46 verläuft, daß sich um 180° voneinander getrennte Linienstränge ergeben, die sich von dem unteren Rand des Hütchens 45 bis zur Stirnfläche 48 des Ansatzes 46 erstrecken. Zur Übertragung der Aufreißkräfte dienen Grifflaschen 53,54, die von der Umfangswand 51 des Hütchens 45 seitwärts abstehen (Fig. 11).

Das mit dem Hütchen 45 ausgestattete Besteck 10 oder 100 wird erst kurz vor der Punktion fertig montiert. Bis dahin sitzt nur das Hütchen 45 auf der Verlängerung bzw. 135 der Hülse 11 bzw. 111. Das Durchstechen des Trokars 12 bzw. 112 durch die Schlitzung 49 in der Stirnwand 47 und das Einstecken eines Katheterschlauches durch die Schlitzung 50 in der Stirnwand 48 machen das Besteck punktionsbereit. Nach Entfernen des Trokars 12 bzw. 112 schließt sich die Stirnwand 47 und der Katheterschlauch kann durch den Ansatz 46 vorgeschoben werden, ohne daß Urin aus dem Besteck heraustritt.

## Ansprüche

1. Besteck zur Punktion eines Körperhohlraumes, insbesondere zur suprapubischen Punktion der Harnblase, bestehend aus einer längsteilbaren Hülse (11), in der ein herausziehbarer Trokar (12) steckt, dessen massives Endstück (13) über den distalen Rand (14) der Hülse (11) vorsteht und mit einer dreikantig angeschliffenen Spitze (41) versehen ist, **dadurch gekennzeichnet**, daß der dreikantige Anschliff an der Spitze (41) einer konischen Dilatierzone (40) des Endstückes (13) des Trokars (12) ausgebildet ist, die aus dem bis an den zylindrischen Umfang (20) des Trokars (12) abfallend angefasten distalen Rand (14) der Hülse (11) hervorragt.

2. Besteck nach Anspruch 1, **dadurch gekennzeichnet**, daß der Trokar (12) hohl ist, hinter der Dilatierzone (40) und vor dem distalen Rand (14) der Hülse (11) ein in den Trokarlängskanal (22) mündendes Seitenloch (19) aufweist und am proximalen Ende mit einem Auslaß (21) versehen ist.

3. Besteck nach Anspruch 1,

**dadurch gekennzeichnet,** daß der Trokar (112) massiv ist und in seiner Außenfläche mindestens eine längsverlaufende Kerbe (60) besitzt, die sich in dem zylindrischen Teil des Trokars (112) von der Dilatierzone (140) bis zu einem Auslaß am proximalen Ende erstreckt.

4. Besteck nach einem der Ansprüche 1 bis 3, **dadurch gekennzeichnet,** daß der angefaste Rand (14) der Hülse (11) schräg zur Längsachse verläuft.

5. Besteck nach einem der Ansprüche 1 bis 3, **dadurch gekennzeichnet,** daß der angefaste Rand (114) der Hülse (111) senkrecht zur Längsachse verläuft.

6. Besteck nach einem der Ansprüche 1 bis 5, **dadurch gekennzeichnet,** daß im Bereich eines Griffstückes (30) des Trokars (12) und eines Griffteiles (18) der Hülse (11) eine Arretiervorrichtung (36,37) zur gegenseitigen Blockierung von Hülse (11) und Trokar (12) vorgesehen ist.

7. Besteck nach einem der Ansprüche 2 bis 6, **dadurch gekennzeichnet,** daß der Auslaß (21) des Trokarlängskanals (22) mit einer transparenten Kammer des Griffstückes (30) des Trokars (12) in Verbindung steht.

8. Besteck nach einem der Ansprüche 3 bis 6, **dadurch gekennzeichnet,** daß der Auslaß der Trokarkerbung (60) mit einer transparenten Kammer des Griffteiles (118) der Hülse (111) in Verbindung steht.

9. Besteck nach Anspruch 7 oder 8, **dadurch gekennzeichnet,** daß die transparente Kammer als an eine Auslaßleitung des Griffstückes (30) oder des Griffteiles (118) angeschlossener transparenter Schlauch (33; 133) mit verschließbarem Ende ausgebildet ist.

10. Besteck nach Anspruch 7 oder 8, **dadurch gekennzeichnet,** daß das Griffstück (30) des Trokars (12) oder das Griffteil (118) der Hülse (111) zur Bildung der transparenten Kammer hohl und aus transparentem Material ausgebildet sind.

11. Besteck nach einem der Ansprüche 1 bis 10, **dadurch gekennzeichnet,** daß der längsteilbare Griffteil (18;118) der Hülse (11;111) ein Dichtelement (38;39) zur Abdichtung der Hülse (11;111) gegenüber dem Trokar (12; 112) aufweist.

12. Besteck nach Anspruch 11, **dadurch gekennzeichnet,** daß das Dichtelement als scheibenförmige Membran (38) oder Hütchen (39) aus Elastomermaterial ausgebildet ist und daß die Membran (38) oder das Hütchen (39) längs mindestens einer Schwächungslinie teilbar ist.

13. Besteck nach Anspruch 11, **dadurch gekennzeichnet,** daß das Dichtelement als Hütchen (45) aus Elastomermaterial mit seitlichem schlauchförmigem Ansatz (46) ausgebildet ist, daß das Hütchen (45) und der seitliche Ansatz (46) längsteilbar sind und daß die Stirnflächen (47; 48) von Hütchen (45) und Ansatz (46) mit einer Schlitzung (49;50) zum Durchlaß des Trokars (22) bzw. eines Katheters versehen sind.

14. Besteck nach einem der Ansprüche 1 bis 13, **dadurch gekennzeichnet,** daß die Hülse (11; 111) aus Metall oder Polymermaterial oder einer Kombination beider Materialien besteht und daß der Trokar (12; 112) aus Metall hergestellt ist.

FIG. 1

FIG. 2

FIG. 3

FIG. 4

FIG. 5

FIG. 6

FIG. 7

FIG. 8

**FIG.9**

**FIG.12**

**FIG.13**

**FIG.10**

**FIG.11**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-U-8 702 954 (H. WILKE) * Seite 4, Zeilen 7-22; Anspruch 1; Figuren 1,3 * | 1,4-5, 14 | A 61 B 17/34 |
| A | | 2-3,6- 13 | |
| | --- | | |
| Y | DE-U-8 506 522 (P. KREBS) * Seite 6, Zeilen 1-4; Seite 13, Zeilen 22-26; Seite 14, Zeilen 11-16; Seite 16, Zeile 22 - Seite 17, Zeile 8; Figuren 4,12 * | 1,4-5, 14 | |
| A | | 2-3,6- 13 | |
| | --- | | |
| A | DE-U-8 616 477 (UROMED KURT DREWS) * Seite 7, Zeilen 10-19; Ansprüche 1,3; Fig. * | 1-2,7- 10 | |
| | --- | | |
| A | US-A-4 561 445 (J.J. BERKE & C.T. MICHAEL) * Zusammenfassung; Spalte 3, Zeilen 10-18; Spalte 5, Zeilen 37-40; Figuren 5-8 * | 1 | RECHERCHIERTE SACHGEBIETE (Int. Cl.5) |
| | --- | | A 61 B |
| A | EP-A-0 135 364 (ENDOTHERAPEUTICS) * Seite 2, Zeilen 1-29; Seite 3, Zeile 27 - Seite 4, Zeile 17; Anspruch 4; Figuren 1,4,5B * | 1,6 | A 61 M |
| | --- | | |
| A,D | DE-A-3 347 150 (BEIERSDORF) * Zusammenfassung; Seite 5, Zeile 26 - Seite 6, Zeile 15; Figuren 1-2 * | 3-4,6 | |
| | --- | | |
| A | EP-A-0 137 061 (J.M. CLARKE) * Ansprüche 1-3; Figuren 1,11 * | 11-13 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 26-03-1990 | NICE P.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)